(19) Europäisches Patentamt
European Patent Office
Office européen des brevets

(11) Veröffentlichungsnummer: **0 273 868 B1**

(12) **EUROPÄISCHE PATENTSCHRIFT**

(45) Veröffentlichungstag der Patentschrift: **08.07.92**

(51) Int. Cl.$^5$: **C10M 133/40**, C10M 135/32, C07D 215/06

(21) Anmeldenummer: **87810780.4**

(22) Anmeldetag: **23.12.87**

Die Akte enthält technische Angaben, die nach dem Eingang der Anmeldung eingereicht wurden und die nicht in dieser Patentschrift enthalten sind.

(54) **N-substituierte Tetrahydrochinoline als Antioxidantien für Schmiermittel.**

(30) Priorität: **30.12.86 CH 5253/86**

(43) Veröffentlichungstag der Anmeldung:
**06.07.88 Patentblatt 88/27**

(45) Bekanntmachung des Hinweises auf die Patenterteilung:
**08.07.92 Patentblatt 92/28**

(84) Benannte Vertragsstaaten:
**DE ES FR GB IT**

(56) Entgegenhaltungen:
**EP-A- 0 072 349**
**US-A- 2 846 435**
**US-A- 3 829 292**
**US-A- 4 025 631**

(73) Patentinhaber: **CIBA-GEIGY AG**
**Klybeckstrasse 141**
**CH-4002 Basel(CH)**

(72) Erfinder: **Meier, Hans Rudolf, Dr.**
**Rte du Confin 54**
**CH-1723 Marly(CH)**
Erfinder: **Evans, Samuel, Dr.**
**Route des Charbonnières 17**
**CH-1723 Marly(CH)**

**EP 0 273 868 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft Schmiermittel-, Hydrauliköl- oder Metallbearbeitungsflüssigkeitszusammensestzungen enthaltend als Stabilisatoren N-substituierte 1,2,3,4-Tetrahydrochinoline, neue N-substituierte 1,2,3,4-Tetrahydrochinoline sowie die Verwendung solcher N-substituierter 1,2,3,4-Tetrahydrochinoline als Stabilisatoren in Schmiermitteln, Hydraulikölen und Metallbearbeitungsflüssigkeiten.

Mineralischen und synthetischen Schmiermitteln, Hydraulikölen oder Metallbearbeitungsflüssigkeiten werden im allgemeinen verschiedene Zusatzstoffe zur Verbesserung ihrer Gebrauchseigenschaften beigegeben. Insbesondere besteht ein Bedarf an Additiven, welche die Oxidation bzw. Alterung des Schmiermittels, des Hydrauliköls oder der Metallbearbeitungsflüssigkeit wirksam inhibieren und somit deren Lebensdauer verlängern.

Aus der EP-A 72,349 sind am N-Atom unsubstituierte 1,2,3,4-Tetrahydrochinoline als Antioxidantien für Schmiermittel bekannt. In der DE-A 2,156,371 sind N-substituierte 1,2,3,4-Tetrahydrochinolinderivate als Stabilsatoren für Farbbildnermassen für druckempfindliche Aufzeichnungen beschrieben. Ferner sind 1-Amino- resp. 1-Hydroxyalkyl-1,2,3,4-tetrahydrochinoline in der US-A-3,247,211 als Ausgangsprodukte für Farbstoffe beschrieben. Weiter sind aus der US-A 2,846,435 N-Alkyl-1,2,2,3-Tetrahydrochinoline als Kautschukstabilisatoren und aus der GB-A 795,497 N-substituierte 1,2,3,4-Tetrahydrochinoline als Flexibilitätsverbesserer für polymere Polyolefinbeschichtungen auf Metallen bekannt.

Gegenstand der vorliegenden Erfindung ist eine Zusammensetzung enthaltend

a) mindestens ein Schmiermittel, ein Hydrauliköl oder eine Metallbearbeitungsflüssigkeit auf der Basis von Mineralöl oder synthetischen Oelen und

b) 0,05 - 5 Gew.-%, bezogen auf das Gesamtgewicht der Schmiermittel-, Hydrauliköl- bzw. Metallbearbeitungsflüssigkeitszusammensetzung, mindestens einer Verbindung der Formeln I, II oder/und V,

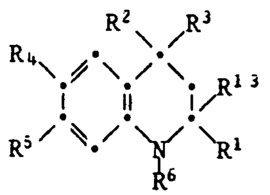

(I)

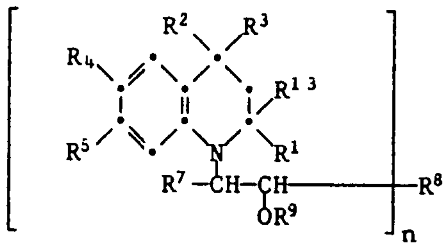

(II)

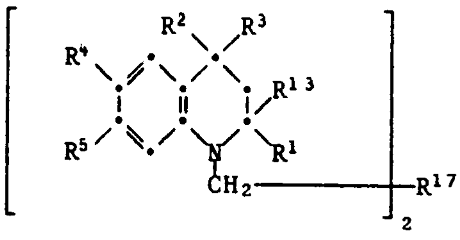

(V)

worin $R^1$ und $R^2$ unabhängig voneinander $C_1$-$C_8$-Alkyl, unsubstituiertes oder durch $C_1$-$C_{12}$-Alkyl, -Cl, -Br, -I, $-NO_2$, -OH oder $C_1$-$C_{12}$-Alkoxy substituiertes Phenyl oder Naphthyl, ferner Benzyl bedeuten, $R^3$ Wasserstoff oder Methyl, $R^4$ und $R^5$ unabhängig voneinander Wasserstoff, Methyl, Methoxy, -Cl oder $-No_2$ bedeuten und $R^4$ zusätzlich in Formel I einen Rest der Formel III

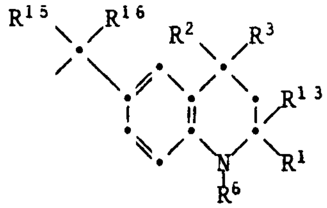

(III)

darstellt, worin $R^{15}$ und $R^{16}$ unabhängig voneinander Wasserstoff, $C_1$-$C_9$-Alkyl, unsubstituiertes oder durch $C_1$-$C_{12}$-Alkyl substituiertes Phenyl, weiter Benzyl, $\alpha$-Methylbenzyl oder $\alpha,\alpha$-Dimethylbenzyl bedeuten, oder $R_{15}$ und $R_{16}$ zusammen mit dem C-Atom, an das sie gebunden sind, einen 5-, 6- oder 7-Ring bilden, mit der Masssgabe, dass, wenn $R^4$ einen Rest der Formel III darstellt, $R^5$ Wasserstoff ist, wieter worin $R^6$ $C_1$-$C_{18}$-Alkyl, $C_3$-$C_{18}$-Alkenyl, $C_7$-$C_{12}$-Aralkyl, $C_7$-$C_{12}$-Alkaryl sowie durch $C_1$-$C_{12}$-Alkyl, -Cl, -Br, -I, $-NO_2$, -OH oder $C_1$-$C_{12}$-Alkoxy kernsubstituiertes Benzyl, $R^7$ Wasserstoff oder Methyl, $R^8$, für den Fall n = 1, Wasserstoff, $-CH_2OR^{14}$, $C_1$-$C_{18}$-Alkyl, Phenyl, $-CH_2SR^{14}$ oder, für den Fall n = 2 bis 4, einen Rest der Formeln

$$-CH_2-O-(CH_2-\underset{R^{10}}{CH}-O)_q-CH_2-,\quad -(CH_2)_m-,\quad -CH_2O-(CH_2)_p-OCH_2-\quad ,$$

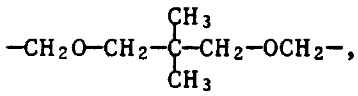 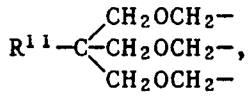 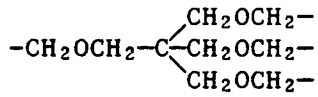

sowie

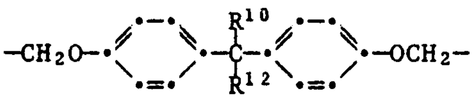

darstellen,
ferner worin $R^9$ Wasserstoff, Methyl,

$$-(CH_2-\underset{R^{10}}{CH}-O)_m-R^{10}$$

oder

$$-\overset{\overset{O}{\|}}{C}-R^6\ ,$$

$R^{10}$, $R_{12}$ und $R^{13}$ unabhängig voneinander Wasserstoff oder Methyl, $R^{11}$ Wasserstoff, Methyl oder Ethyl und $R^{14}$ $C_1$-$C_{18}$-Alkyl, $C_7$-$C_{13}$-Alkaryl, Phenyl, Naphthyl, Pentalyl, Heptalyl, Indacyl oder unsubstituiertes oder durch $C_1$-$C_{12}$-Alkyl, -Cl, -Br, -I, $-NO_2$, -OH oder $C_1$-$C_{12}$-Alkoxly kernsubstituiertes Benzyl bedeuten, und n die Zahlen 1 bis 4, m und q die Zahlen 1 bis 10 und p die Zahlen 2 bis 10 darstellen und $R^{17}$ entweder $C_xH_{2x}$, wobei x gleich 0 bis 10 ist, oder -CH=CH- darstellt.

Stellen $R^1$ und $R^2$ $C_1$-$C_8$-Alkyl dar, so handelt es sich um geradkettige oder verzweigte Alkylreste, wie beispielsweise Methyl, Ethyl, n-Propyl, Isopropyl, n-Butyl, Isobutyl, sec.-Butyl, tert.-Butyl, geradkettiges oder verzweigtes Pentyl, Hexyl, Heptyl oder Octyl.

Stellen $R^{15}$ und $R^{16}$ $C_1$-$C_9$-Alkyl dar, so handelt es sich um geradkettige oder verzweigte Alkylreste, wie beispielsweise Methyl, Ethyl, n-Propyl, Isopropyl, n-Butyl, Isobutyl, sec.-Butyl, tert.-Butyl, geradkettiges oder

verzweigtes Pentyl, Hexyl, Heptyl, Octyl oder Nonyl.

Stellen $R^6$, $R^8$ und $R^{14}$ $C_1$-$C_{18}$-Alkyl dar, so handelt es sich um geradkettige oder verzweigte Alkylreste, wie beispielsweise Methyl, Ethyl, n-Propyl, Isopropyl, n-Butyl, Isobutyl, sec.-butyl, tert.-Butyl, geradkettiges oder verzweigtes Pentyl, Hexyl, Heptyl, Octyl, Nonyl, Decyl, Undecyl, Dodecyl, Tridecyl, Tetradecyl, Pentadecyl, Hexadecyl, Heptadecyl oder Octadecyl. Bevorzugt ist $R^8$ $C_1$-$C_{12}$-Alkyl.

Stellt $R^6$ $C_3$-$C_{18}$-Alkenyl dar, so handelt es sich um geradkettige oder verzweigte Alkenylreste, die eine oder mehrere, bevorzugt jedoch eine Doppelbindung enthalten, wie beispielsweise Allyl, n-Butenyl, 1,3-Butadienyl, i-Pentenyl, Pentenyl, Hexenyl, Heptenyl, Octenyl, Nonenyl, Decenyl, Undecenyl, Dodecenyl, Tridecenyl, 2-Nonyl-2-butenyl, Tetradecenyl, pentadecenyl, hexadecenyl, 8-Heptadecenyl, 2-Octadecenyl oder Oleyl. Bevorzugt sind Allyl oder Oleyl, insbesondere Allyl.

Stellen $R^1$, $R^2$, $R^{15}$ oder $R^{16}$ durch $C_1$-$C_{12}$-Alkyl substituiertes Phenyl und $R^6$ oder $R^{14}$ durch $C_1$-$C_{12}$-Alkyl kernsubstituiertes Benzyl dar, so können der Phenyl- bzw. Benzylrest ein- oder mehrfach, bevorzugt ein- bis zweifach, für $R^{15}$ und $R^{16}$ jedoch nur einfach substituiert sein. Bei $C_1$-$C_{12}$-Alkyl handelt es sich beispielsweise um Methyl, Ethyl, n-Propyl, Isopropyl, n-Butyl, Isobutyl, sec.-Butyl, tert.-Butyl, geradkettiges oder verzweigtes Nonyl oder Dodecyl.

Stellen $R^1$ und $R^2$ durch $C_1$-$C_{12}$-Alkoxy substituiertes Phenyl und $R^6$ oder $R^{14}$ durch $C_1$-$C_{12}$-Alkoxy kernsubstituiertes Benzyl dar, so können der Phenyl- bzw. Benzylrest ein- oder mehrfach, bevorzugt jedoch ein- bis zweifach, substituiert sein; bei $C_1$-$C_{12}$-Alkoxy handelt es sich beispielsweise um Methoxy, Ethoxy, n-Propoxy, Isopropoxy, n-Butoxy, Isobutoxy, sec.-Butoxy, tert.-butoxy, geradkettiges oder verzweigtes Nonyloxy oder Dodecyloxy.

Stellt $R^6$ $C_7$-$C_{12}$-Aralkyl, bevorzugt Phenyl-$C_1$-$C_4$-Alkyl, dar, so handelt es sich beispielsweise um Benzyl, 1- oder 2-Phenethyl, 3-Phenylpropyl, $\alpha,\alpha$-Dimethylbenzyl, 2-Phenylisopropyl, 2-Phenylhexyl oder Naphthylmethyl. Bevorzugt sind Benzyl und $\alpha,\alpha$-Dimethylbenzyl, insbesondere Benzyl.

Stellen $R^6$ $C_7$-$C_{12}$-Alkaryl und $R^{14}$ $C_7$-$C_{13}$-Alkaryl dar, so handelt es sich beim Arylrest um Phenyl oder Naphthyl, bevorzugt jedoch um Phenyl, die ein- oder mehrfach, vorzugsweise jedoch ein- oder zweifach, durch $C_1$-$C_6$-Alkyl resp. $C_1$-$C_7$-Alkyl, bevorzugt durch $C_1$-$C_4$-Alkyl, substituiert sind, wie beispielsweise 2,4,6-Trimethylphenyl, 2,5-Dimethylphenyl, 4-Isopropylphenyl, 4-Hexylphenyl, 4-Methylphenyl, 3,4-Diethylphenyl, 2-Methylnaphthyl oder 2,6-Dimethylnaphthyl. Bevorzugt sind jedoch 2,5-Dimethylphenyl oder 4-Methylphenyl, insbesondere 4-Methylphenyl.

Bevorzugt ist eine Zusammensetzung enthaltend als Komponente b) mindestens eine Verbindung der Formeln I oder/und II, worin in den Verbindungen der Formeln I bzw. II $r^1$ oder $R^2$ unabhängig voneinander $C_1$-$C_8$-Alkyl oder Benzyl, besonders bevorzugt $C_1$-$C_8$-Alkyl, ganz speziell bevorzugt $C_1$-$C_4$-Alkyl und insbesondere bevorzugt Methyl oder Ethyl bedeuten.

Ebenfalls bevorzugt ist eine Zusammensetzung enthaltend als Komponente b) mindestens eine Verbindung der Formeln I oder/und II, worin $R^3$ Wasserstoff ist.

Weiter von Interesse ist eine Zusammensetzung enthaltend als Komponente b) mindestens eine Verbindung der Formeln I oder/und II, worin $R^4$ und $R^5$ unabhängig voneinander Wasserstoff oder Methyl bedeuten.

Von besonderem Interesse ist eine Zusammensetzung enthaltend als Komponente b) mindestens eine Verbindung der Formel I, worin $R^4$ einen Rest der Formel III

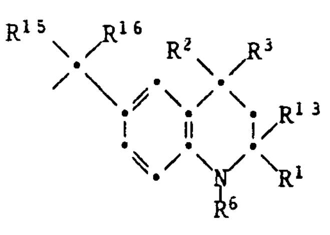

(III)

bedeutet.

Ebenfalls interessant ist eine Zusammensetzung enthaltend als Komponente b) mindestens eine Verbindung der Formel I, worin $R^6$ $C_1$-$C_{12}$-Alkyl, $C_3$-$C_8$-Alkenyl oder Benzyl, bevorzugt jedoch $C_1$-$C_4$-Alkyl, Allyl, Methallyl oder Benzyl bedeutet.

Auch interessant ist eine Zusammensetzung enthaltend als Komponente b) mindestens eine Verbindung der Formel II, worin $R^7$ Wasserstoff ist.

Eine weitere Ausführungsform ist eine Zusammensetzung enthaltend als Komponente b) mindestens eine Verbindung der Formel II, worin $R^8$ Wasserstoff, $C_1$-$C_4$-Alkyl, Phenyl,

$-CH_2O-(CH_2)_p-OCH_2-$ ,

$-CH_2O-(CH_2-CH_2-O)_q-CH_2-$, $-(CH_2)_m-$,

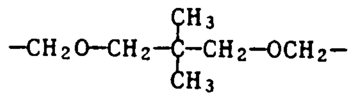 oder 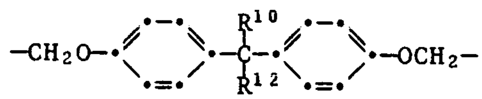 ,

bevorzugt jedoch Wasserstoff oder Methyl, und n 1 oder 2 bedeuten.

Eine spezielle Ausführungsform ist eine Zusammensetzung enthaltend als Komponente b) mindestens eine Verbindung der Formel II, worin $R^9$ Wasserstoff, Methyl,

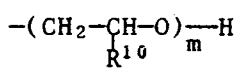

oder

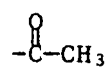 ,

bevorzugt jedoch Wasserstoff, bedeutet.

Eine ebenfalls interessante Ausführungsform ist eine Zusammensetzung enthaltend als Komponente b) mindestens eine Verbindung der Formeln I oder/und II, worin $R^{13}$ Methyl ist.

Eine besondere Ausführungsform ist eine Zusammensetzung enthaltend als Komponente b) mindestens eine Verbindung der Formel II, worin n 1 oder 2 ist.

Eine zusätzliche Ausführungsform ist eine Zusammensetzung enthaltend als Komponente b) mindestens eine Verbindung der Formel II worin m, p ode q unabhängig voneinander die Zahlen 2 bis 6 darstellen.

Ein weiterer Gegenstand der vorliegenden Erfindung sind die neuen Verbindungen N-(2-Hydroxypropyl)-2,2,4,7-tetramethyl-1,2,3,4-tetrahydrochinolin, N-[2-Hydroxy-3-(tert.-nonylthio)-propyl]-2,2,4-trimethyl-1,2,3,4-tetrahydrochinolin, N-[2-Hydroxy-3-(tert.-nonylthio)-propyl]-2,2,4,6-tetramethyl-1,2,3,4 - tetrahydrochinolin.

Ebenfalls Gegenstand der vorliegenden Erfindung sind die neuen Verbindungen der Formel IV

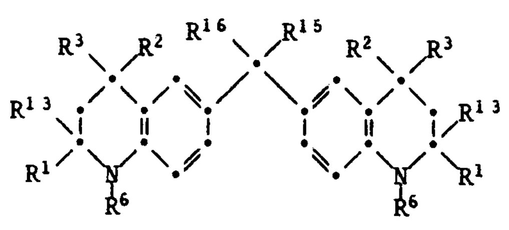

(IV)

worin $R^1$ und $R^2$ unabhängig voneinander $C_1$-$C_8$-Alkyl, unsubstituiertes oder durch $C_1$-$C_2$-Alkyl, -Cl, -Br, -I, $-NO_2$, -OH oder $C_1$-$C_{12}$-Alkoxy substituiertes Phenyl oder Naphthyl, ferner Benzyl bedeuten, $R^3$ Wasserstoff oder Methyl ist, $R^6$ $C_1$-$C_{18}$-Alkyl, $C_3$-$C_8$-Alkenyl, $C_7$-$C_{12}$-Aralkyl, $C_7$-$C_{12}$-Alkaryl sowie durch $C_1$-$C_{12}$-Alkyl, -Cl, -Br, -J, $-NO_2$, -OH oder $C_1$-$C_{12}$-Alkoxy kernsubstituiertes Benzyl bedeutet, $R^{13}$ Wasserstoff oder Methyl ist und $R^{15}$ und $R^{16}$ unabhängig voneinander Wasserstoff, $C_1$-$C_9$-Alkyl, unsubstituiertes oder durch $C_1$-$C_{12}$-Alkyl substituiertes Phenyl, weiter Benzyl, $\alpha$-Methylbenzyl oder $\alpha,\alpha$-Dimethylbenzyl darstellen, oder worin $R^{15}$ und $R^{16}$ zusammen mit dem C-Atom, an das sie gebunden sind, einen 5-, 6- oder 7-Ring bilden.

Speziell bevorzugt sind die Verbindungen der Formel IV, woring $R^{15}$ und $R^{16}$ zusammen mit dem C-Atome, an das sie gebunden sind, einen 5-, 6- oder 7-Ring bilden, oder $R^{15}$ und $R^{16}$ unabhängig voneinander Wasserstoff oder $C_1$-$C_4$-Alkyl darstellen.

Beispiele für Verbindungen der Formel I und II sind:

1,2,2,4-Tetramethyl-1,2,3,4-tetrahydrochinolin

1-Ethyl-2,2,4-trimehtyl-1,2,3,4-tetrahydrochinlin
1,2-Dimethyl-2,4-diethyl-1,2,3,4-tetrahydrochinolin
2-Methyl-1,2,4-triethyl-1,2,3,4-tetrahydrochinolin
1-Benzyl-2,2,4-trimethyl-1,2,3,4-tetrahydrochinolin
1-Allyl-2,2,4-trimethyl-1,2,3,4-tetrahydrochinolin
1-Benzyl-2,2,4,6,7-pentamethyl-1,2,3,4-tetrahydrochinolin
1-Butyl-2,2,4-trimethyl-1,2,3,4-tetrahydrochinolin
1-(2-Hydroxethyl)-2,2,4-trimethyl-1,2,3,4-tetrahydrochinolin
1-(2-Hydroxypropyl)-2,2,4-trimethyl-1,2,3,4-tetrahydrochinolin
1-(2-Acetoxypropyl)-2,2,4,6-tetramethyl-1,2,3,4-tetrahydrochinolin
1-(2-Hydroxyethyl)-2,2,4,6-tetramethyl-1,2,3,4-tetrahydrochinolin
1-(2-Hydroxyethyl)-2,4-diethyl-2-methyl-1,2,3,4-tetrahydrochinolin
1-(2-Hydroxyethyl)-2,2,4,7-tetramethyl-1,2,3,4-tetrahydrochinolin
1-(2-Hydroxypropyl)-2,4-diethyl-2-methyl-1,2,3,4-tetrahydrochinolin
1-(2-Acetoxyethyl)-2,2,4-trimethyl-1,2,3,4-tetrahydrochinolin
1-(β-Methallyl)-2,2,4-trimethyl-1,2,3,4-tetrahydrochinolin
1,16-Bis-[2,2,4-trimethyl-1,2,3,4-tetrahydrochinol-1-yl]-2,15-dihydroxy-4,13-dioxa-hexadecan
1,16-Bis-[2,2,4-trimethyl-1,2,3,4-tetrahydrochinol-1-yl]-2,15-dihydroxy-4,7,10,13-tetraoxa-hexadecan
1,14-Bis-[2,2,4-trimethyl-1,2,3,4-tetrahydrochinol-1-yl]-2,13-dihydroxy-4,11-dioxa-tetradecan
1,13-Bis-[2,2,4-trimethyl-1,2,3,4-tetrahydrochinol-1-yl]-2,12-dihydroxy-4,7,10-trioxa-tridecan
1,10-Bis-[2,4-diethyl-2-methyl-1,2,3,4-tetrahydrochinol-1-yl]-2,9-dihydroxy-4,7-dioxa-decan
2,2-Di-{4-[3′-(2,2,4-trimethyl-1,2,3,4-tetrahydrochinol-1-yl)-2′-hydroxy-prop-1′-oxy]-phenyl}-propan
1,1-Di-{4-[3′(2,2,4,6-tetramethyl-1,2,3,4-tetrahydrochinol-1-yl)-2′-hydroxy-prop-1′-oxy]-phenyl}-methan
1-[2,2,4-trimethyl-1,2,3,4-tetrahydrochinol-1-yl)-3-(tert.-octyl-thio)-propan-2-ol
1-[2,2,4-trimethyl-1,2,3,4-tetrahydrochinol-1-yl)-3-(p-tolyloxy)-propan-2-ol
1-[2,2,4-trimethyl-1,2,3,4-tetrahydrochinol-1-yl)-3-(o-tolyloxy)-propan-2-ol
1-(4-Butylphenyl)-2,4-diethyl-6,7-dimethoxy-2-methyl-1,2,3,4-tetrahydrochinolin
1-(Oleyl)-2,4-diphenyl-4-methyl-1,2,3,4-tetrahydrochinolin
1-(3-Methoxy-pentadec-2-yl)-4-benzyl-6,7-dichlor-2,4-dimethyl-1,2,3,4-tetrahydrochinolin
1,1-Bis-[1-(3′,5′-dimethyl-phenyl)-2,2,4,-trimethyl-1,2,3,4-tetrahydrochinol-6-yl]-methan
1,1-Bis-{1-(2′-hydroxyethyl)-2,4-diethyl-2-methyl-1,2,3,4-tetrahydrochinol-6-yl]-methan
1,1-Bis-[1,2-diethyl-2,4-dimethyl-1,2,3,4-tetrahydrochinol-6-yl]-cyclohexan.

Die Herstellung der Verbindungen der Formeln I oder II erfolgt nach an sich bekannter Weise.

Beispielsweise können Anilin und ein Methylketon im molaren Verhältnis 1:2, wie in Org. Synthesis, Coll. Vol. III, S. 329-32, oder in der US-A 2 846,435 beschrieben, umgesetzt werden. Das daraus resultierende N-unsubstituierte 1,2,3,4-Tetrahydrochinolin kann nach literaturbekannten, dem Fachmann geläufigen Methoden mit üblichen Alkylierungs-, Arylierungs- oder Benzylierungsmitteln wie z.B. Dimethylsulfat, Allylbromid, Jodbenzol oder Benzylchlorid zu den Verbindungen der Formel I umgesetzt werden.

Die Verbindungen der Formel II können nach der in der US-A 3,247,211 beschriebenen Weise hergestellt werden.

Neue Verbindungen der Formel I oder II können auf analoge Weise hergestellt werden.

Die erfindungsgemässen Verbindungen der Formeln I, II und IV sind als Antioxidantien für Schmiermittel, Hydrauliköle und Metallbearbeitungsflüssigkeiten, insbesondere für Schmiermittel und Hydrauliköle und ganz speziell für Schmiermittel, sehr gut geeignet.

Ein weiterer Gegenstand der vorliegenden Erfindung ist deshalb auch die Verwendung der Verbindungen der Formeln I, II und IV als Antioxidantien für Schmiermittel, Hydrauliköle oder Metallbearbeitungsflüssigkeiten.

Die Verbindungen der Formeln I und II sind in Schmiermitteln, Hydraulikölen und Metallbearbeitungsflüssigkeiten in ausreichender Menge löslich und werden diesen Substraten bevorzugt in einer Menge von 0,1 - 2,5 Gew.-%, bezogen auf das Gesamtgewicht der Schmiermittel-, Hydrauliköl- oder Metallbearbeitungsflüssigkeitszusammensetzung zugesetzt.

Solche Schmiermittel-, Hydrauliköl- und Metallbearbeitungsflüssigkeitssysteme können von polarer oder unpolarer Natur sein. Die Auswahlkriterien ergeben sich aus den Löslichkeitseigenschaften der entsprechenden Verbindungen.

Die in Frage kommenden Schmiermittel, Hydraulikflüssigkeiten bzw. Metallbearbeitungsflüssigkeiten sind dem Fachmann geläufig und z.B. im "Schmiermitel Taschenbüch: (Hüthig Verlag, Heidelberg, 1974) resp. in "Ullmanns Encyclopädie der technischen Chemie", Bd. 13, Seiten 85-94 (Verlag Chemie, Weinheim, 1977) bzw. in D. Klamann "Schmierstoffe und verwandte Produkte" Seiten 158-174 (Verlag Chemie,

Weinheim, 1982) beschrieben.

Besonders geeignet sind neben Mineralölen z.B. Poly-α-Olefine, Schmiermittel auf Esterbasis, Phosphate, Glykole, Polyglykole und Polyalkenglykole, sowie deren Mischungen mit Wasser.

Die Schmiermittel, Hydraulikflüssigkeiten oder Metallbearbeitungsflüssigkeiten können zusätzlich andere Additive enthalten, die zugegeben werden, um die Grundeigenschaften dieser Substanzen noch weiter zu verbessern; dazu gehören: weitere antioxidantien, Metallpassivatoren, Rostinhibitoren, Viskositätsindex-Verbesser, Stockpunkterniedriger, Dispergiermittel Detergentien, sowie Hochdruck-Zusätze und Antiverschleiss-Additive.

Beispiele für phenolische Antioxidantien

1. Alkylierte Monophenole

2,6-Di-tert-butyl-4-methylphenol
2,6-Di-tert-butylphenol
2-tert-Butyl-4,6-dimethylphenol
2,6-Di-tert-butyl-4-ethylphenol
2,6-Di-tert-butyl-4-n-butylphenol
2,6-Di-tert-butyl-4-iso-butylphenol
2,6-Di-cyclopentyl-4-methylphenol
2-(α-Methylcyclohexyl)-4,6-dimethylphenol
2,6-Di-octadecyl-4-methylphenol
2,4,6-Tri-cyclohexylphenol
2,6-Di-tert-butyl-4-methoxymethlphenol
o-tert-Butylphenol

2. Alkylierte Hydrochinone

2,6-Di-tert-butyl-4-methoxyphenol
2,5-Di-tert-butyl-hydrochinon
2,5-Di-tert-amyl-hydrochinon
2,6-Diphenyl-4-octadecyloxyphenol

3. Hydroxylierte Thiodiphenylether

2,2′-Thio-bis-(6-tert-butyl-4-methylphenol)
2,2′-Thio-bis-(4-octylphenol)
4,4′-Thio-bis-(6-tert-butyl-3-methylphenol)
4,4′-Thio-bis-(6-tert-butyl-2-methylphenol)

4. Alkyliden-Bisphenole

2,2′-Methylen-bis-(6-tert-butyl-4-methylphenol)
2,2′-Methylen-bis-(6-tert-butyl-4-ethylphenol)
2,2′-Methylen-bis-[4-methyl-6-(α-methylcyclohexyl)-phenol]
2,2′-Methylen-bis-(4-methyl-6-cyclohexylphenol)
2,2′-Methylen-bis-(6-nonyl-4-methylphenol)
2,2′-Methylen-bis-(4,6-di-tert-butylphenol)
2,2′-Ethyliden-bis-(4,6-di-tert-butylphenol)
2,2′-Ethyliden-bis-(6-tert-butyl-4-iso-butylphenol
2,2′-Methylen-bis-[6-(α-methylbenzyl)-4-nonylphenol]
2,2′-Methylen-bis-[6-(α,α-dimethylbenzyl)-4-nonylphenol]
4,4′-Methylen-bis-(2,6-di-tert-butylphenol)
4,4′-Methylen-bis-(6-tert-butyl-2-methylphenol)
1,1-Bis-(5-tert-butyl-4-hydroxy-2-methylphenyl)-butan
2,6-Di-(3-tert-butyl-5-methyl-2-hydroxybenzyl)-4-methylphenol
1,1,3-Tris-(5-tert-butyl-4-hydroxy-2-methylphenyl)-3-n-dodecylmecaptobutan
Ethylenglycol-bis-[3,3-bis-(3′-tert-butyl-4′-hydroxyphenyl)-butyrat]

Di-(3-tert-butyl-4-hydroxy-5-methylphenyl)-dicyclopentadien
Di-[2-(3′-tert-butyl-2′-hydroxy-5′-methyl-benzyl)-6-tert-butyl-4-methyl-phenyl]-terephthalat.

5. Benzylverbindungen

1,3,5-Tri-(3,5-di-tert-butyl-4-hydroxybenzyl)-2,4,6-trimethylbenzol
Di-(3,5-di-tert-butyl-4-hydroxybenzyl)-sulfid
3,5-Di-tert-butyl-4-hydroxybenzyl-mercaptoessigsäure-isooctylester
Bis-(4-tert-butyl-3-hydroxy-2,6-dimethylbenzyl)-dithiol-terephthalat
1,3,5-Tris-(3,5-di-tert-butyl-4-hydroxybenzyl)-isocyanurat
1,3,5-Tris-(4-tert-butyl-3-hydroxy-2,6-dimethylbenzyl)-isocyanurat
3,5-Di-tert-butyl-4-hydroxybenzyl-phosphonsäure-dioctadecylester
3,5-Di-tert-butyl-4-hydroxybenzyl-phosphonsäure-monoethylester-Calcium-salz.

6. Acylaminophenole

4-Hydroxy-laurinsäureanilid
4-Hydroxy-stearinsäureanild
2,4-Bis-octylmercapto-6-(3,5-di-tert-butyl-4-hydroxyanilino)-s-triazin
N-(3,5-di-tert-butyl-4-hydroxyphenyl)-carbaminsäureoctylester.

7. Ester der β-(3,5-Di-tert-butyl-4-hydroxyphenyl)-propionsäure

mit ein- oder mehrwertigen Alkoholen, wie z.B. mit

| | |
|---|---|
| Methanol | Diethylenglycol |
| Octadecanol | Triethylenglycol |
| 1,6-Hexandiol | Pentaerythrit |
| Neopentylglycol | Tris-hydroxyethyl-isocyanurat |
| Thiodiethylenglycol | Di-hydroxyethyl-oxalsäurediamid |

8. Ester der β(5-tert-butyl-4-hydroxy-3-methylphenyl)-propionsäure

mit ein- oder mehrwertigen Alkoholen, wie z.B. mit

| | |
|---|---|
| Methanol | Diethylenglycol |
| Octadecanol | Triethylenglycol |
| 1,6-Hexandiol | Pentaerythrit |
| Neopentylglycol | Tris-hydroxyethyl-isocyanurat |
| Thiodiethylenglycol | Di-hydroxyethyl-oxalsäurediamid |

9. Amide der β-(3,5-Di-tert-butyl-4-hydroxyphenyl)-propionsäure,

wie z.B.
N,N′-Di-(3,5-di-tert-butyl-4-hydroxyphenylpropionyl)-hexamethylendiamin
N,N′-Di-(3,5-di-tert-butyl-4-hydroxyphenylpropionyl)-trimethylendiamin
N,N′-Di-(3,5-di-tert-butyl-4-hydroxyphenylpropionyl)-hydrazin.

Beispiele für aminische Antioxidantien:

N,N′-Di-isopropyl-p-phenylendiamin
N,N′-Di-sec-butyl-p-phenylendiamin
N,N′-Bis(1,4-dimethyl-pentyl)-p-phenylendiamin
N,N′-Bis(1-ethyl-3-methyl-pentyl)-p-phenylendiamin

N,N′-Bis(1-methyl-heptyl)-p-phenylendiamin
N,N′-Diphenyl-p-phenylendiamin
N,N′-Di-(naphthyl-2)-p-phenylendiamin
N-Isopropyl-N′-phenyl-p-phenylendiamin
N-(1,3-Dimethyl-butyl)-N′-phenyl-p-phenylendiamin
N-(1-Methyl-heptyl)-N′-phenyl-p-phenylendiamin
N-Cyclohexyl-N′-phenyl-p-phenylendiamin
4-(p-Toluol-sulfonamido)-diphenylamin
N,N′-Dimethyl-N,N′-di-sec-butyl-p-phenylendiamin
Diphenylamin
4-Isopropoxy-diphenylamin
N-Phenyl-1-naphthylamin
N-Phenyl-2-naphthylamin
octyliertes Diphenylamin
4-n-Butylaminophenol
4-Butyrylamino-phenol
4-Nonanoylamino-phenol
4-Dodecanoylamino-phenol
4-Octadecanoylamino-phenol
Di-(4-methoxy-phenyl)-amin
2,6-Di-tert-butyl-4-dimethylamino-methyl-phenol
2,4′-Diamino-diphenylmethan
4,4′-Diamino-diphenylmethan
N,N,N′,N′-Tetramethyl-4,4′-diamino-diphenylmethan
1,2-Di-[2-methyl-phenyl)-amino]-ethan
1,2-Di-phenylamino)-propan
(o-Tolyl)-biguanid
Di-[4-(1′,3′-dimethyl-butyl)-phenyl)amin
tert-octyliertes N-Phenyl-1-naphthylamin
Gemisch aus mono- und dialkylierten tert-Butyl-/tert-Octyldiphenylaminen.

Beispiele für Metallpassivatoren sind:

für Kupfer, z.B.:
Triazol, Benztriazol und deren Derivate, 2-Mercaptobenzthiazol, 2,5-Dimercaptothiadiazol, Salicyliden-propylendiamin, Salze von Salicylaminoguanidin.

Beispiele für Rost-Inhibitoren sind:

a) Organische Säuren, ihre Ester, Metallsalze und Anhydride, z.B.:
N-Oleoyl-sarcosin, Sorbitan-mono-oleat, Blei-naphthenat, Dodecenylbernsteinsäure-anhydrid, Alkenylbernsteinsäure-Halbester, 4-Nonylphenoxy-essigsäure.
b) Stickstoffhaltige Verbindungen, z.B.:
I. Primäre, sekundäre oder tertiäre aliphatische oder cycloaliphatische Amine und Amin-Salze von organischen und anorganischen Säuren, z.B. öllösliche Alkylammoniumcarboxylate.
II. Heterocyclische Verbindungen, z.B.:
Substituierte Imidazoline und Oxazoline.
c) Phosphorhaltige Verbindungen, z.B.:
Aminsalze von Phosphorsäurepartialestern.
d) Schwefelhaltige Verbindungen, z.B.:
Barium-dinonylnaphthalin-sulfonate, Calciumpetroleum-sulfonate.

Beispiele für Viskositätsindex-Verbesserer sind z.B.

Polymethacrylate, Vinylpyrrolidon/Methacrylat-Copolymere, Polybutene, Olefin-Copolymere, Styrol/Acrylat-Copolymer.

Beispiele für Stockpunkterniedriger sind z.B.:

Polymethacrylat, alkylierte Naphthalinderivate.

Beispiele für Dispergiermittel/Tenside sind z.B.:

Polybutenylbernsteinsäure-imide, Polybutenylphosphonsäurederivate, basische Magnesium-, Calcium, und Bariumsulfonate und -phenolate.

Beispiele für Verschleissschutz-Additive sind z.B.:

Schwefel und/oder Phosphor und/oder Halogen enthaltende Verbindungen, wie geschwefelte pflanzliche Oele, Zinkdialkyldithiophosphate, Tritolyl-phosphat, chlorierte Paraffine, Alkyl- und Arylsulfide.

Beispiel 1: 350 g 2,2,4-Trimethyl-1,2,3,4-tetrahydrochinolin, 2000 ml Ethanol und 110 g Ethylenoxid werden in einem Autoklaven während 12 Stunden auf 180°C erhitzt. Danach wird das Ethanol abdestilliert und das kristallisierte 1-(2-Hydroxyethyl)-2,2,4-trimethyl-1,2,3,4-tetrahydrochinolin abfiltriert. Nach dem Umkristallisieren aus Ethanol wird ein Produkt mit dem Smp. 73-75°C erhalten.

Analog zu Beispiel 1 werden die Beispiele 2-6 hergestellt.

Beispiel 7: 52,6 g 2,2,4-Trimethyl-1,2,3,4-tetrahydrochinolin und 37,2 g Methanphosphonsäuredimethylester werden zusammen unter leichtem Stickstoffstrom währen 5 Stunden auf 160°C erhitzt. Danach wird das Reaktionsgemisch auf 60°C abgekühlt und in 100 ml Toluol aufgenommen und mit 130 g NaOH-Lösung (10 %) alkalisch gestellt. Die organische Phase wird abgetrennt, zweimal mit je 100 ml Wasser gewaschen und am Rotationsverdampfer bei 80°C eingedampft. Anschliessend wird der Rückstand am Hochvakuum bei 60°C getrocknet. Es werden 54,1 g eines klaren, leicht braunen Oels mit einem Siedepunkt von 274°C bei 1013 mbar erhalten.

Die Beispiele 8 bis 11 werden auf analoge Weise erhalten.

Beispiel 12: 35,1 g 2,2,4-Trimethyl-1,2,3,4-tetrahydrochinolin werden in einem Kolben vorgelegt, auf 90°C erwärmt und unter Rühren 47,6 g tert.-Nonylglycidylthioether eingetropft. Nach beendeter Zugabe wird das Gemisch noch 0,5 Stunden bei 90°C nachgerührt. Es wird ein Oel vom Sdp. 185°C bei 0,03 mbar erhalten.

Beispiel 13 wird analog hergestellt.

Tabelle 1

| Beispiel Nr. | Verbindung | Smp./Sdp. |
|---|---|---|
| 1 | 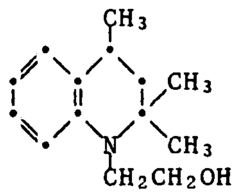 | 73- 75°C |
| 2 | 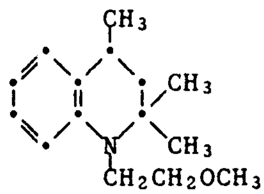 | 110-112°C/ 0,4 mbar |
| 3 | 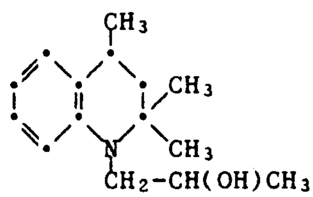 | 130-134°C/ 0,53 mbar |
| 4 | 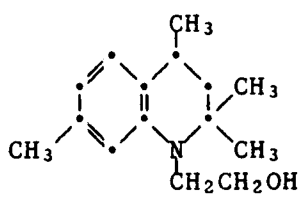 | 78- 79°C |
| 5 | 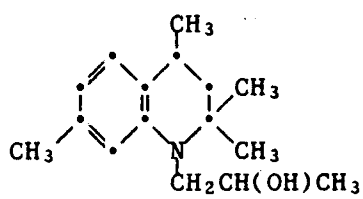 | 120°C/ 0,2 mbar |
| 6 | 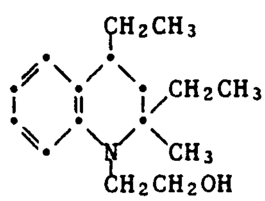 | 130-135°C/ 0,13 mbar |

| Beispiel Nr. | Verbindung | Smp./Sdp. |
|---|---|---|
| 7 | 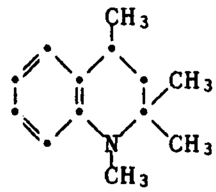 | 274°C/ 1013 mbar |
| 8 | 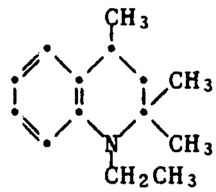 | 84- 86°C/ 0,2 mbar |
| 9 | 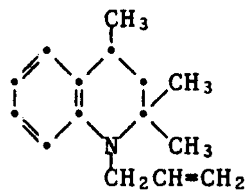 | 283°C/ 1013 mbar |
| 10 | 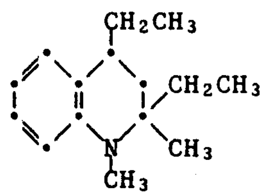 | 105°C/ 0,27 mbar |
| 11 | 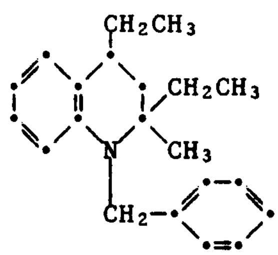 | 296°C/ 1013 mbar |
| 12 | 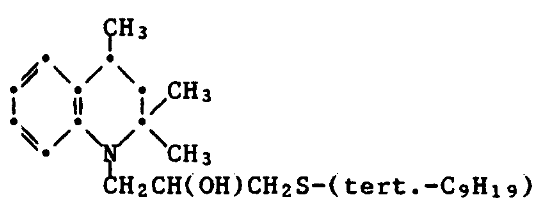 | 185°C/ 0,03 mbar |

| Beispiel Nr. | Verbindung | Smp./Sdp. |
|---|---|---|
| 13 | 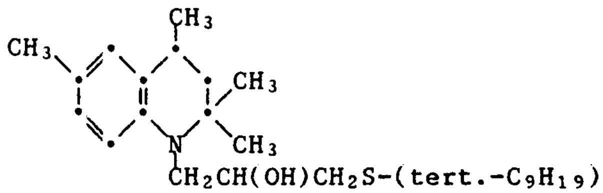 | 194°C/ 0,03 mbar |

Beispiel 14:

TFOUT-Test(Thin Film Oxygen Uptake Test)

Dieser Test ist eine modifizierte Version des "Rotary Bomb Oxidationstests für Mineralöle" (ASTM D 2272). Er wird genau beschrieben in "C.S. Ku und S.M. Hsu, A Thin-Film Oxygen Uptake Test for the Evaluation of Automotive Crankcase Lubricants, Lubrication Engineering, Vol. 40 (2), 75-83 (1984)". Als Testöl dient ein Motorenöl auf Mineralölbasis, welches die Hälfte der üblichen Menge an Zinkdithiophosphat (0,75 %; Zinkgehalt 0,06 %, bezogen auf das Motorenöl) enthält.

Die hergestellte Verbindung wird im beschriebenen Motorenöl in Gegenwart von 2 % Wasser, einer flüssigen oxidierten nitrierten Fraktion eines Motorenbenzins als Katalysator (4 % Einsatzkonzentration) und eines flüssigen Metallnaphthenates als weiterer Katalysator (4 % Einsatzkonzentration) getestet. Wasser und die beiden flüssigen Katalysatorsubstanzen werden unter der No. Standard Reference Material 1817 vom National Bureau of Standards (NBS) mit Analysen-Zertifikat geliefert. Der Versuch ist bei einem deutlichen Knick des Druck/Zeit-Diagramms beendet. Die in der Tabelle 2 angegebenen Resultate bedeuten die Zeit (in Minuten) bis zum Knick des Druck-Zeit-Diagramms

Lange Zeiten ensprechen guter Stabilisatorwirksamkeit. Konzentration des Stabilisators: 0,5 Gew.-%, bezogen auf das Oel.

Tabelle 2

| Stabilisator | Minuten bis deutlicher Druckabfall |
|---|---|
| keiner | 76 |
| Beispiel 1 | 199 |
| Beispiel 3 | 185 |
| Beispiel 4 | 197 |
| Beispiel 6 | 186 |
| Beispiel 7 | 220 |
| Beispiel 8 | 218 |
| Beispiel 9 | 200 |

## Patentansprüche

**1.** Zusammensetzung enthaltend

a) mindestens ein Schmiermittel, ein Hydraliköl oder eine Metallbearbeitungsflüssigkeit auf der Basis von Mineralöl oder synthetischen Oelen und

b) 0,05 - 5 Gew.-%, bezogen auf das Gesamtgewicht der Schmiermittel-, Hydrauliköl- bzw. Metallbearbeitungsflüssigkeitszusammensetzung, mindestens einer Verbindung der Formeln I, II oder/und V,

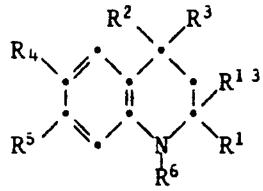

(I)

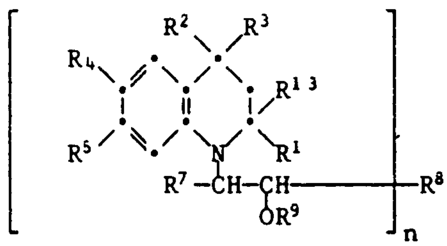

(II)

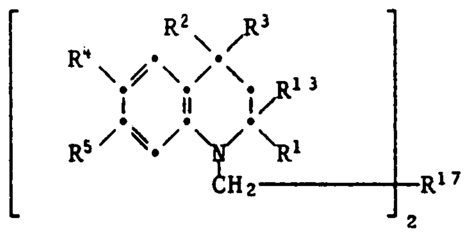

(V)

worin $R^1$ und $R^2$ unabhängig voneinander $C_1$-$C_8$-Alkyl, unsubstituiertes oder durch $C_1$-$C_{12}$-Alkyl, -Cl, -Br, -I, $-NO_2$, -OH oder Ci1-$C_{12}$-Alkoxy substituiertes Phenyl oder Naphthyl, ferner Benzyl bedeuten, $R^3$ Wasserstoff oder Methyl, $R^4$ und $R^5$ unabhängig voneinander Wasserstoff, Methyl, Methoxy, -Cl oder $-NO_2$ bedeuten und $R^4$ zusätzlich in Formel I einen Rest der Formel III

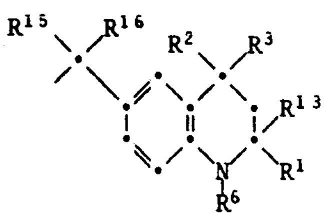

(III)

darstellt, worin $R^{15}$ und $R^{16}$ unabhängig voneinander Wasserstoff, $C_1$-$C_9$-Alkyl, unsubstituiertes oder durch $C_1$-$C_{12}$-Alkyl substitiuiertes Phenyl, weiter Benzyl, $\alpha$-Methylbenzyl oder $\alpha,\alpha$-Dimethylbenzyl bedeuten, oder $R_{15}$ und $R_{16}$ zusammen mit dem C-Atom, and das sie gebunden sind, einen 5-, 6- oder 7-Ring bilden, mit der Massgabe, dass, wenn $R^4$ einen Rest der Formel III darstellt, $R^5$ Wasserstoff ist, weiter worin $R^6$ $C_1$-$C_{18}$-Alkyl, $C_3$-$C_{18}$-Alkenyl, $C_7$-$C_{12}$-Aralkyl, $C_7$-$C_{12}$-Alkaryl sowie durch $C_1$-$C_{12}$-Alkyl, -Cl, -Br, -H, $-NO_2$, -OH oder $C_1$-$C_{12}$-Alkoxy kernsubstituiertes Benzyl, $R^7$ Wasserstoff oder Methyl, $R^8$, für den Fall n = 1, Wasserstoff, $-CH_2OR^{14}$, $C_1$-$C_{18}$-Alkyl, Phenyl, $-CH_2SR^{14}$ oder, für den Fall n = 2 bis 4, einen Rest der Formeln

$$-CH_2-O-(CH_2-\underset{R^{10}}{CH}-O)_q-CH_2-,\quad -(CH_2)_m-,\quad -CH_2O-(CH_2)_p-OCH_2-\ ,$$

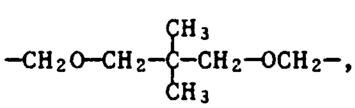, 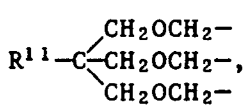, 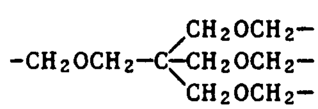

sowie

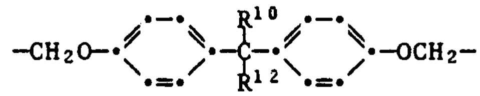

darstellen,
ferner worin $R^9$ Wasserstoff, Methyl,

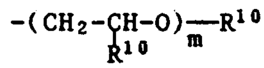

oder

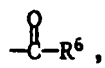

$R^{10}$, $R^{12}$ und $R^{13}$ unabhängig voneinander Wasserstoff oder Methyl, $R^{11}$ Wasserstoff, Methyl oder Ethyl und $R^{14}$ $C_1$-$C_{18}$-Alkyl, $C_7$-$C_{13}$-Alkaryl, Phenyl, Naphthyl, Pentalyl, Heptalyl, Indacyl oder unsubstituiertes oder durch $C_1$-$C_{12}$-Alkyl, -Cl, -Br, -I, $-NO_2$, -OH oder $C_1$-$C_{12}$-Alkoxy kernsubstituiertes Benzyl bedeuten, und n die Zahlen 1 bis 4, m und q die Zahlen 1 bis 10 und p die Zahlen 2 bis 10 darstellen und worin $R^{17}$ entweder $C_xH_{2x}$, wobei x gleich 0 bis 10 ist, oder -CH = CH-darstellt.

2. Zusammensetzung gemäss Anspruch 1, worin in den Verbindungen der Formeln I oder/und II $R^1$ oder $R^2$ unabhängig voneinander $C_1$-$C_4$-Alkyl bedeuten.

3. Zusammensetzung gemäss Anspruch 2, worin in den Verbindungen der Formeln I oder/und II $R^1$ oder $R^2$ unabhängig voneinander Methyl oder Ethyl bedeuten.

4. Zusammensetzung gemäss Anspruch 1, worin in den Verbindungen der Formeln I oder/und II $R^3$ Wasserstoff ist.

5. Zusammensetzung gemäss Anspruch 1, worin in den Verbindungen der Formeln I oder/und II $R^4$ oder $R^5$ unabhängig voneinander Wasserstoff oder Methyl bedeuten.

6. Zusammensetzung gemäss Anspruch 1, worin in den Verbindungen der Formel I $R^4$ einen Rest der Formel III

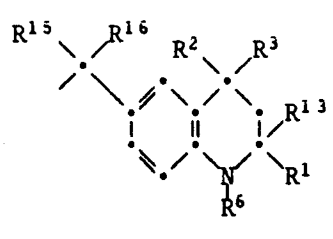

(III)

bedeutet.

7. Zusammensetzung gemäss Anspruch 1, worin in den Verbindungen der Formel I $R^6$ $C_1$-$C_{12}$-Alkyl, $C_3$-$C_8$-Alkenyl und vorzugsweise $C_1$-$C_4$-Alkyl, Allyl, Methallyl oder Benzyl bedeutet.

8. Zusammensetzung gemäss Anspruch 1, worin in den Verbindungen der Formel I $R^2$ = Methyl, $R^3$ Wasserstoff, $R^1$ und $R^{13}$ Methyl, $R^4$ und $R^5$ Wasserstoff und $R^6$ $-CH_3$, $-CH-CH=CH_2$, $-CH_2CH_2OH$,

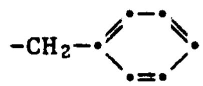

oder

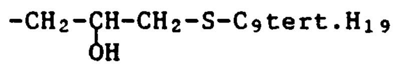

bedeuten.

**9.** Zusammensetzung gemäss Anspruch 1, worin in den Verbindungen der Formel II $R^7$ Wasserstoff ist.

**10.** Zusammensetzung gemäss Anspruch 1, worin in den Verbindungen der Formel II $R^8$ Wasserstoff, $C_1$-$C_4$-Alkyl, Phenyl,

$$-CH_2O-(CH_2-CH_2-O)_q-CH_2-,\quad -(CH_2)_m-,\quad -CH_2O(CH_2)_p-OCH_2-,$$

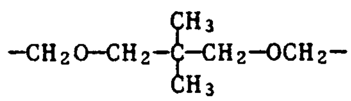 oder 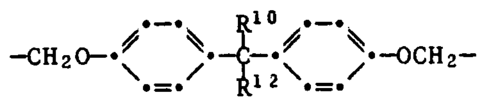

und n 1 oder 2 bedeuten und vorzugsweise in den Verbindungen der Formel II $R^8$ Wasserstoff oder Methyl ist.

**11.** Zusammensetzung gemäss Anspruch 1, worin in den Verbindungen der Formel II $R^9$ Wasserstoff, Methyl,

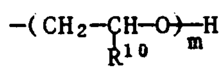

oder

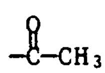

bedeutet.

**12.** Zusammensetzung gemäss Anspruch 1, worin in den Verbindungen der Formeln I oder/und II $R^{13}$ Methyl ist.

**13.** Zusammensetzung gemäss Anspruch 1, worin in den Verbindungen der Formel II n 1 oder 2 ist.

**14.** Zusammensetzung gemäss Anspruch 1, worin in den Verbindungen der Formel II m, p oder q unabhängig voneinander die Zahlen 2 bis 6 darstellen.

**15.** Zusammensetzung gemäss Anspruch 1, worin die Komponente a) ein Schiermittel oder ein Hydrauliköl bedeutet.

**16.** N-(2-Hydroxypropyl)-2,2,4,7-tetramehtyl-1,2,3,4-tetrahydrochinolin, N-[2-Hydroxy-3-(tert.-nonylthio)-propyl]-2,2,4-trimethyl-1,2,3,4-tetrahydrochinolin, N-[2-Hydroxy-3-(tert.-nonylthio)-propyl]-2,2,4,6-tetramehtyl-1,2,3,4 -tetrahydrochinolin.

**17.** Verbindungen der Formel IV

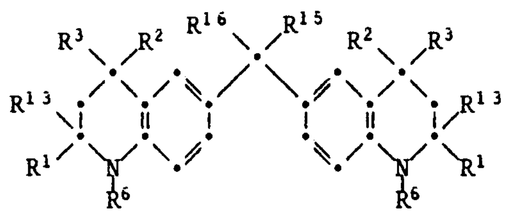

(IV)

worin $R^1$ und $R^2$ unabhängig voneinander $C_1$-$C_8$-Alkyl, unsubstituiertes oder durch $C_1$-$C_{12}$-Alkyl, -Cl, -Br, -I, -$NO_2$, -OH oder $C_1$-$C_{12}$-Alkoxy substituiertes Phenyl oder Naphthyl, ferner Benzyl bedeuten, $R^3$ Wasserstoff oder Methyl ist, $R^6$ $C_1$-$C_{18}$-Alkyl, $C_3$-$C_8$-Alkenyl, $C_7$-$C_{12}$-Aralkyl, $C_7$-$C_{12}$-Alkaryl sowie durch $C_1$-$C_{12}$-Alkyl, -Cl, -Br, -J, -$NO_2$, -OH oder $C_1$-$C_{12}$-Alkoxy kernsubstituiertes Benzyl bedeutet, $R^{13}$ Wasserstoff oder Methyl ist und $R^{15}$ und $R^{16}$ unabhängig voneinander Wasserstoff, $C_1$-$C_9$-Alkyl, unsubstituiertes oder durch $C_1$-$C_{12}$-Alkyl substituiertes Phenyl, weiter Benzyl, $\alpha$-Methylbenzyl oder $\alpha,\alpha$-Dimethylbenzyl darstellen, oder worin $R^{15}$ und $R^{16}$ zusammen mit dem C-Atom, an das sie gebunden sind, einen 5-, 6- oder 7-Ring bilden.

**18.** Verbindungen gemäss Anspruch 17, worin $R^{15}$ und $R^{16}$ zusammen mit dem C-Atom, an das sie gebunden sind, einen 5-, 6- oder 7-Ring bilden, oder $R^{15}$ und $R^{16}$ unabhängig voneinander Wasserstoff oder $C_1$-$C_4$-Alkyl darstellen.

**19.** Verwendung der Verbindungen der Formeln I, II, IV und V nach Anspruch 1 als Antioxidantien für Schmiermittel Hydrauliköle oder Metallbearbeitungsflüssigkeiten.

## Claims

**1.** A composition containing

a) at least one lubricant, hydraulic oil or metal-working fluid based on mineral oil or synthetic oils and

b) 0.05-5 % by weight, based on the total weight of the lubricant, hydraulic oil or metal-working fluid composition, of at least one compound of the formulae I, II or/and V

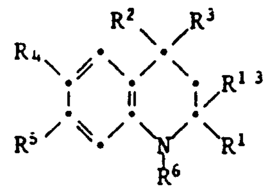

(I)

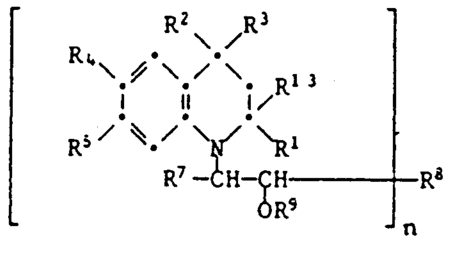

(II)

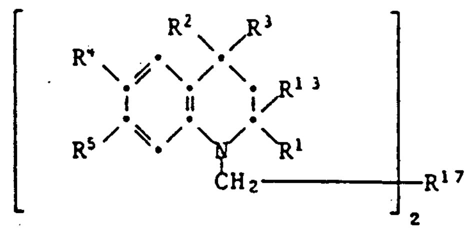

(V)

in which $R^1$ and $R^2$ are independently of each other $C_1$-$C_8$alkyl, unsubstituted or $C_1$-$C_{12}$alkyl-, Cl-, Br-, I-, $NO_2$-, OH- or $C_1$-$C_{12}$alkoxy- substituted phenyl or naphthyl, or benzyl, $R^3$ is hydrogen or methyl, $R^4$ and $R^5$ are independently of each other hydrogen, methyl, methoxy, -Cl or -$NO_2$ and $R^4$ additionally in the formula I is a radical of the formula III

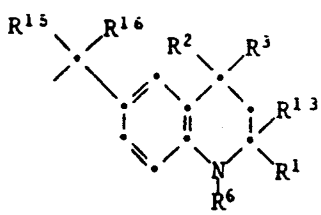

(III)

in which $R^{15}$ and $R^{16}$ are independently of each other hydrogen, $C_1$-$C_9$alkyl, unsubstituted or $C_1$-$C_{12}$alkyl-substituted phenyl, or benzyl, $\alpha$-methylbenzyl or $\alpha,\alpha$-dimethylbenzyl, or $R^{15}$ and $R^{16}$ are together with the C atom to which they are bonded a 5-, 6- or 7-ring, with the proviso that when $R^4$ is a radical of the formula III $R^5$ is hydrogen, and in which furthermore $R^6$ is $C_1$-$C_{18}$alkyl, $C_3$-$C_{18}$alkenyl, $C_7$-$C_{12}$aralkyl, $C_7$-$C_{12}$alkaryl or $C_1$-$C_{12}$alkyl, Cl-, Br-, I-, $NO_2$-, OH- or $C_1$-$C_{12}$alkoxy-ringsubstituted benzyl, $R^7$ is hydrogen or methyl, $R^8$, if n is l, is hydrogen, -$CH_2OR^{14}$, $C_1$-$C_{18}$alkyl, phenyl or -$CH_2SR^{14}$ or, if n is 2 to 4, a radical of the formulae

$$-CH_2-O-(CH_2-\underset{R^{10}}{CH}-O)_q-CH_2-,\quad -(CH_2)_m-,\quad -CH_2O-(CH_2)_p-OCH_2-\ ,$$

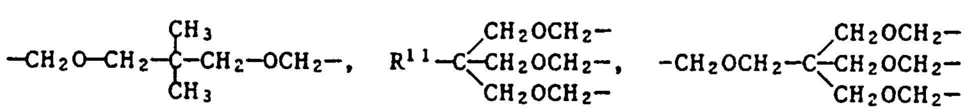

and

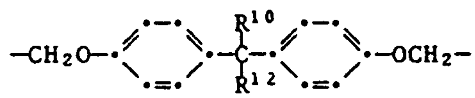

and in which furthermore $R^8$ if hydrogen, methyl,

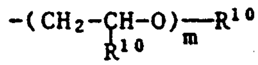

or

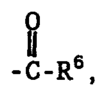

$R^{10}$, $R^{12}$ and $R^{13}$ are independently of one another hydrogen or methyl, $R^{11}$ is hydrogen, methyl or ethyl and $R^{14}$ is $C_1$-$C_{18}$alkyl, $C_7$-$C_{13}$alkaryl, phenyl, naphthyl, pentalyl, heptalyl, indacyl or unsubstituted or $C_1$-$C_{12}$alkyl-, Cl-, Br-, I-, $NO_2$-, OH- or $C_1$-$C_{12}$alkoxy- ringsubstituted benzyl, n is a number from 1 to 4, m and q are numbers from 1 to 10, p is a number from 2 to 10, and $R^{17}$ is either $C_xH_{2x}$, where x is 0 to 10, or -CH-CH-.

2. A composition according to claim 1, wherein, in the compounds of the formulae I or/and II, $R^1$ or $R^2$ are independently of each other $C_1$-$C_4$alkyl.

3. A composition according to claim 2, wherein, in the compounds of the formulae I or/and II, $R^1$ or $R^2$ are independently of each other methyl or ethyl.

4. A composition according to claim 1, wherein, in the compounds of the formula I or/and II, $R^3$ is hydrogen.

5. A composition according to claim 1, wherein, in the compounds of the formula I or/and II, $R^4$ or $R^5$ are independently of each other hydrogen or methyl.

6. A composition according to claim 1, wherein, in the compounds of the formula I $R^4$ is a radical of the formula III

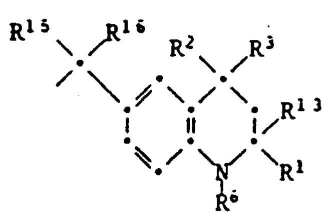

(III)

7. A composition according to claim 1, wherein, in the compounds of the formula I $R^6$ is $C_1$-$C_{12}$alkyl, $C_3$-$C_8$alkenyl or preferably $C_1$-$C_4$alkyl, allyl, methallyl or benzyl.

8. A composition according to claim 1, wherein, in the compounds of the formula I $R^2$ is methyl, $R^3$ is hydrogen, $R^1$ and $R^{13}$ are methyl. $R^4$ and $R^5$ are hydrogen and $R^6$ is $-CH_3$, -CH-CH-$CH_2$, $-CH_2CH_2OH$,

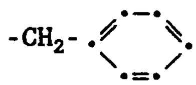

or

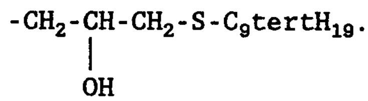

9. A composition according to claim 1, wherein, in the compounds of the formula II, $R^7$ is hydrogen.

10. A composition according to claim 1, wherein, in the compounds of the formula II, $R^8$ is hydrogen, $C_1$-$C_4$alkyl, phenyl,

$$-CH_2O-(CH_2-CH_2-O)_q-CH_2-,\quad -(CH_2)_m-,\quad -CH_2O(CH_2)_pOCH_2-,$$

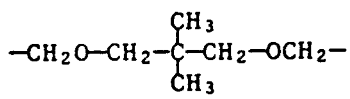 or 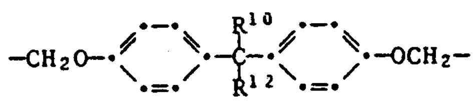

and n is 1 or 2 and, preferably, in the compounds of the formula II $R^8$ is hydrogen or methyl.

11. A composition according to claim 1, wherein, in the compounds of the formula II, $R^9$ is hydrogen, methyl,

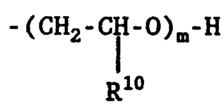

or

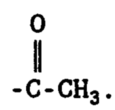

12. A composition according to claim 1, wherein, in the compounds of the formulae I or/and II, $R^{13}$ is methyl.

13. A composition according to claim 1, wherein, in the compounds of the formula II, n is 1 or 2.

14. A composition according to claim 1, wherein, in the compounds of the formula II m, p or q are independently of one another a number from 2 to 6.

15. A composition according to claim 1, wherein component a) is a lubricant or a hydraulic oil.

16. N-(2-Hydroxypropyl)-2,2,4,7-tetramethyl-1,2,3,4-tetrahydroquinoline, N-[2-hydroxy-3-(tert-nonylthio)-propyl]-2,2,4-trimethyl-1,2,3,4-tetrahydroquinoline, N-[2-hydroxy-3-(tert-nonylthio)propyl]-2,2,4,6-tetramethyl-1,2,3,4-tetrahydroquinoline.

**17.** A compound of the formula IV

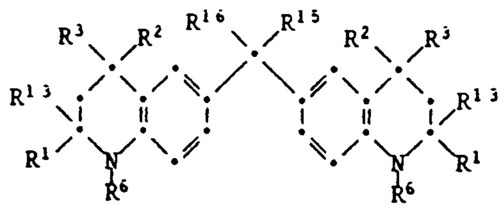

(IV)

in which $R^1$ and $R^2$ are independently of each other $C_1$-$C_8$alkyl, substituted or $C_1$-$C_{12}$alkyl, Cl-, Br-, I-, $NO_2$-, OH- or $C_1$-$C_{12}$alkoxy-un-substituted phenyl or naphthyl, or benzyl, $R^3$ is hydrogen or methyl, $R^5$ is $C_1$-$C_{18}$alkyl, $C_3$-$C_8$alkenyl, $C_7$-$C_{12}$aralkyl, $C_7$-$C_{12}$alkaryl or $C_1$-$C_{12}$alkyl, Cl-, Br-, I-, $NO_2$-, OH- or $C_1$-$C_{12}$alkoxy-ringsubstituted benzyl, $R^{13}$ is hydrogen or methyl, $R^{15}$ and $R^{16}$ are independently of each other hydrogen, $C_1$-$C_9$alkyl, unsubstituted or $C_1$-$C_{12}$alkyl-substituted phenyl, benzyl, $\alpha$-methylbenzyl or $\alpha,\alpha$-dimethylbenzyl, or in which $R^{15}$ and $R^{16}$ together with the C atom to which they are bonded are a 5-, 6- or 7-ring.

**18.** A compound according to claim 18, wherein $R^{15}$ and $R^{16}$ together with the C atom to which they are bonded are a 5-, 6- or 7-ring, or $R^{15}$ and $R^{16}$ are independently of each other hydrogen or $C_1$-$C_4$alkyl.

**19.** A method of using a compound of the formula I, II or IV according to claim 1 as an antioxidant in lubricants, hydraulic oils or metal-working fluids.

**Revendications**

**1.** Composition qui contient :

a) au moins un lubrifiant, une huile hydraulique ou un liquide pour l'usinage de métaux à base d'une huile minérale ou d'huiles synthétiques et

b) de 0,05 à 5 % en poids, par rapport au poids total de la composition pour lubrifiant, huile hydraulique ou liquide pour l'usinage de métaux, d'au moins un composé répondant à l'une des formules I, II et V :

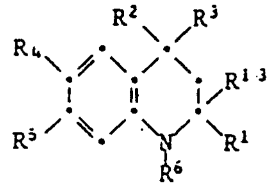

(I)

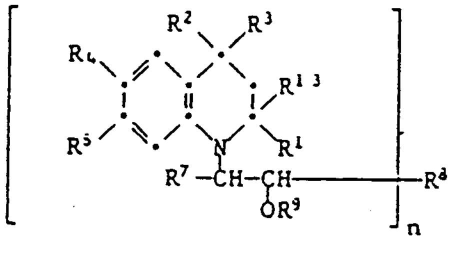

(II)

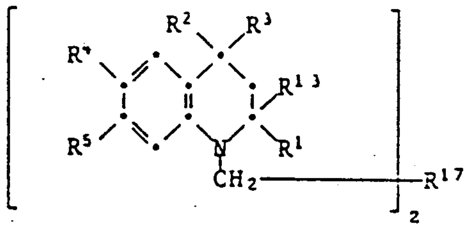

(V)

dans lesquelles

$R^1$ et $R^2$ représentent chacun, indépendamment l'un de l'autre, un alkyle en $C_1$-$C_8$, un radical phényle ou naphtyle non substitué ou porteur d'un alkyle en $C_1$-$C_{12}$, d'un -Cl, d'un -Br, d'un -I, d'un $-NO_2$, d'un -OH ou d'un alcoxy en $C_1$-$C_{12}$, ou encore un benzyle,

$R^3$ représente l'hydrogène ou un méthyle,

$R^4$ et $R^5$ représentent chacun, indépendamment l'un de l'autre, l'hydrogène, un méthyle, un méthoxy, un -Cl ou un $-NO_2$, et $R^4$ peut en outre, dans la formule I, représenter un radical répondant à la formule III :

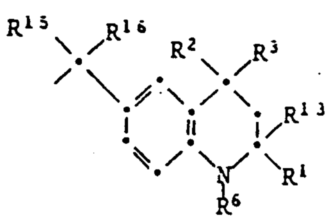

(III)

dans laquelle $R^{15}$ et $R^{16}$ représentent chacun, indépendamment l'un de l'autre, l'hydrogène, un alkyle en $C_1$-$C_9$, un phényle non substitué ou porteur d'un alkyle en $C_1$-$C_{12}$, un benzyle, un $\alpha$-méthylbenzyle ou un $\alpha,\alpha$-diméthylbenzyle, ou $R^{15}$ et $R^{16}$ forment ensemble, et avec l'atome de carbone auquel ils sont liés, un cycle à 5, à 6 ou à 7 maillons, avec la condition que, lorsque $R^4$ représente un radical de formule III, $R^5$ représente l'hydrogène,

$R^6$ représente un alkyle en $C_1$-$C_{18}$, un alcényle en $C_3$-$C_{18}$, un aralkyle en $C_7$-$C_{12}$, un alkylaryle en $C_7$-$C_{12}$ ou encore un benzyle éventuellement porteur, sur son noyau, d'un alkyle en $C_1$-$C_{12}$, d'un -Cl, d'un -Br, d'un -I, d'un $-NO_2$, d'un -OH ou d'un alcoxy en $C_1$-$C_{12}$,

$R^7$ représente l'hydrogène ou un méthyle,

$R^8$ représente, dans le cas où n est égal à 1, l'hydrogène, un radical $-CH_2OR^{14}$, un alkyle en $C_1$-$C_{18}$, un phényle ou un radical $-CH_2SR^{14}$, ou, dans le cas où n est égal à 2, à 3 ou à 4, un radical répondant à l'une des formules suivantes :

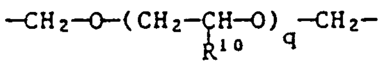, $-(CH_2)_m-$, $-CH_2O-(CH_2)_p-OCH_2-$ ,

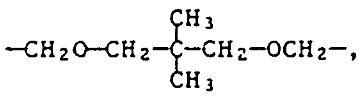, 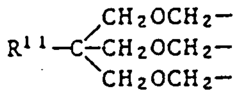, 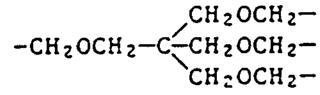

et

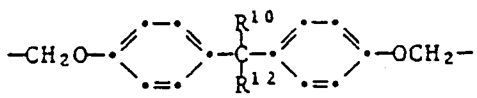

$R^9$ représente l'hydrogène, un méthyle, un radical

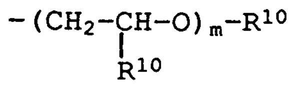

ou un radical

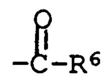,

$R^{13}$ ainsi que $R^{10}$ et $R^{12}$ représentent chacun, indépendamment l'un de l'autre, l'hydrogène ou un méthyle,

et $R^{11}$ représente l'hydrogène, un méthyle ou un éthyle, $R^{14}$ un alkyle en $C_1$-$C_{18}$, un alkylaryle en $C_7$-$C_{13}$, un phényle, un naphtyle, un pentalényle, un heptalényle, un indacényle ou un benzyle non substitué ou porteur, sur son noyau, d'un alkyle en $C_1$-$C_{12}$, d'un -Cl, d'un -Br, d'un -I, d'un $-NO_2$, d'un -OH ou d'un alcoxy en $C_1$-$C_{12}$, n un nombre de 1 à 4, m et q désignent chacun un nombre de 1 à 10, p désigne un nombre de 2 à 10 et $R^{17}$ représente soit un radical $C_xH_{2x}$ dans lequel x désigne un nombre de 0 à 10, soit un radical -CH=CH-.

**2.** Composition selon la revendication 1 caractérisée en ce que, dans les composés de formules I et/ou II, $R^1$ et $R^2$ représentent chacun, indépendamment l'un de l'autre, un alkyle contenant de 1 à 4 atomes de carbone.

**3.** Composition selon la revendication 2 caractérisée en ce que, dans les composés de formules I et/ou II, $R^1$ et $R^2$ représentent chacun, indépendamment l'un de l'autre, un méthyle ou un éthyle.

**4.** Composition selon la revendication 1 caractérisée en ce que, dans les composés de formules I et/ou II, $R^3$ représente l'hydrogène.

**5.** Composition selon la revendication 1 caractérisée en ce que, dans les composés de formules I et/ou II, $R^4$ et $R^5$ représentent chacun, indépendamment l'un de l'autre, l'hydrogène ou un méthyle.

**6.** Composition selon la revendication 1 caractérisée en ce que, dans les composés de formule I, le symbole $R^4$ représente un radical de formule III:

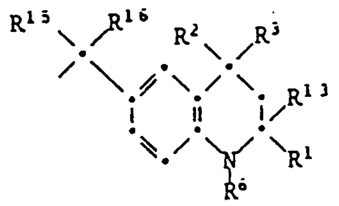

(III).

**7.** Composition selon la revendication 1 caractérisée en ce que, dans les composés de formule I, le symbole $R^6$ représente un alkyle en $C_1$-$C_{12}$ ou un alcényle en $C_3$-$C_8$, de préférence un alkyle en $C_1$-$C_4$, un allyle ou, un méthallyle, ou un benzyle.

**8.** Composition selon la revendication 1 caractérisée en ce que, dans les composés de formule I, le symbole $R^2$ représente un méthyle, $R^3$ représente l'hydrogène, $R^1$ et $R^{13}$ représentent chacun un méthyle, $R^4$ et $R^5$ représentent chacun l'hydrogène et $R^6$ représente un radical $-CH_3$, $-CH_2-CH=CH_2$, $-CH_2CH_2OH$,

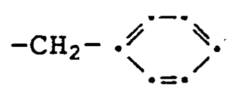

ou

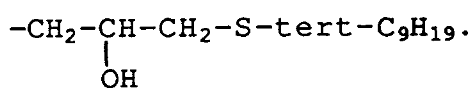

**9.** Composition selon la revendication 1 caractérisée en ce que, dans les composés de formule II, le symbole $R^7$ représente l'hydrogène.

**10.** Composition selon la revendication 1 caractérisée en ce que, dans les composés de formule II, le symbole $R^8$ représente l'hydrogène, un alkyle en $C_1$-$C_4$, un phényle ou un radical

$$-CH_2O-(CH_2-CH_2-O)_q-CH_2-, \quad -(CH_2)_m-, \quad -CH_2O(CH_2)_pOCH_2-,$$

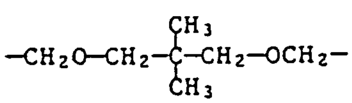 ou 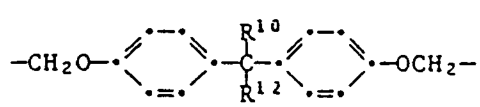

et n est égal à 1 ou à 2, ou mieux, dans les composés de formule II, $R^8$ représente l'hydrogène ou un méthyle.

**11.** Composition selon la revendication 1 caractérisée en ce que, dans les composés de formule II, le symbole $R^9$ représente l'hydrogène, un méthyle, un radical

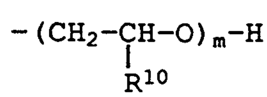

ou un radical

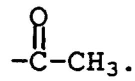

12. Composition selon la revendication 1 caractérisée en ce que, dans les composés de formules I et/ou II, le symbole $R^{13}$ représente un méthyle.

13. Composition selon la revendication 1 caractérisée en ce que, dans les composés de formule II, n est égal à 1 ou à 2.

14. Composition selon la revendication 1 caractérisée en ce que, dans les composés de formule II, m, p et q sont égaux chacun, indépendamment l'un de l'autre, à un nombre de 2 à 6.

15. Composition selon la revendication 1 dans laquelle la composante a) est un lubrifiant ou une huile hydraulique.

16. N(2-Hydroxypropyl)-2,2,4,7-tétraméthyl-1,2,3,4-tétrahydro-quinoléine, N-[2-hydroxy-3-(tert-nonylthio)-propyl]-2,2,4-triméthyl-1,2,3,4-tétrahydro-quinoléine et N-[2-hydroxy-3-(tert-nonylthio)-propyl]-2,2,4,6-tétraméthyl-1,2,3,4-tétrahydro-quinoléine.

17. Composés répondant à la formule IV :

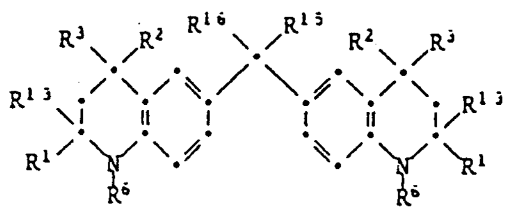

(IV)

dans laquelle

| | |
|---|---|
| $R^1$ et $R^2$ | représentent chacun, indépendamment l'un de l'autre, un alkyle en $C_1$-$C_8$, un radical phényle ou naphtyle non substitué ou porteur d'un alkyle en $C_1$-$C_{12}$, d'un -Cl, d'un -Br, d'un -I, d'un -$NO_2$, d'un -OH ou d'un alcoxy en $C_1$-$C_{12}$, ou encore un benzyle, |
| $R^3$ | représente l'hydrogène ou un méthyle, |
| $R^6$ | représente un alkyle en $C_1$-$C_{18}$, un alcényle en $C_3$-$C_8$,un aralkyle en $C_7$-$C_{12}$, un alkylaryle en $C_7$-$C_{12}$, ou un benzyle porteur, sur son noyau, d'un alkyle en $C_1$-$C_{12}$, d'un -Cl, d'un -Br, d'un -I, d'un -$NO_2$, d'un -OH ou d'un alcoxy en $C_1$-$C_{12}$, |
| $R^{13}$ | représente l'hydrogène ou un méthyle et |
| $R^{15}$ et $R^{16}$ | représentent chacun, indépendamment l'un de l'autre, l'hydrogène, un alkyle en $C_1$-$C_9$, un phényle non substitué ou porteur d'un alkyle en $C_1$-$C_{12}$, un benzyle, un $\alpha$-méthylbenzyle ou un $\alpha,\alpha$-diméthylbenzyle, ou encore $R^{15}$ et $R^{16}$ forment ensemble, et avec l'atome de carbone auquel ils sont liés, un cycle à 5, à 6 ou à 7 maillons. |

18. Composés selon la revendication 17 dans laquelle $R^{15}$ et $R^{16}$ forment ensemble, et avec l'atome de carbone auquel ils sont liés, un cycle à 5, à 6 ou à 7 maillons, ou $R^{15}$ et $R^{16}$ représentent chacun, indépendamment l'un de l'autre, l'hydrogène ou un alkyle en $C_1$-$C_4$.

19. Application des composés de formules I, II, IV et V selon la revendication 1 comme anti-oxydants pour des lubrifiants, des huiles hydrauliques ou des liquides pour l'usinage de métaux.